# EUROPEAN PATENT APPLICATION

(11) **EP 3 598 962 A1**
(43) Date of publication of application: **29.01.2020**
(21) Application number: 19188725.6
(22) Date of filing: 26.07.2019
(51) Int. Cl.: A61H 5/00, A61M 21/00, A61N 1/04, A61N 1/20, A61N 1/36

(54) **SYSTEM FOR TRAINING VISUAL ACUITY**

(30) Priority: 27.07.2018 TW 107126123
(71) Applicant: EYESON TECH LTD, Taichung City 40465 (TW); ASIA UNIVERSITY, Taichung City 41354 (TW)
(72) Inventor: LEE, Shin-Da, 40465 Taichung City (TW); HSU, Chen-Chao, 40465 Taichung City (TW); CHEN, Hsin-Chin, 70163 Tainan City (TW); WANG, Chung-Hui, 40465 Taichung City (TW)
(74) Representative: Mewburn Ellis LLP

(57) **Abstract**

A system for training visual acuity includes an audio player (2) and a controller (6) . The audio player (2) includes a database (21) which stores plural pieces of music, and a headphone (22) with which a user listens to the pieces of music so as to stimulate a visual cortex of the user, where each of the pieces of music contains two audio frequency components that have a frequency difference therebetween ranging from 1 to 45 Hz. The controller (6) is electrically connected to the audio player (2), and is configured to control the audio player (2) to play the pieces of music.

## Description

The disclosure relates to a system for training visual acuity.

Most approaches of vision training, such as focusing at far or eye exercise, aim to train or relax ocular muscles of a subject, while other parts of the visual system (e.g., functions of optic nerve and visual cortex in the visual nerve system) of the subject are not targeted by the training.

Therefore, an object of the disclosure is to provide a system for training visual acuity that can alleviate at least one of the drawbacks of the prior art.

According to the disclosure, the system includes an audio player and a controller. The audio player includes a database which stores plural pieces of music, and a headphone with which a user listens to the pieces of music so as to stimulate a visual cortex of the user, where each of the pieces of music contains two audio frequency components that have a frequency difference therebetween ranging from 1 to 45 Hz. The controller is electrically connected to the audio player, and is configured to control the audio player to play the pieces of music.

Other features and advantages of the disclosure will become apparent in the following detailed description of the embodiments with reference to the accompanying drawings, of which:
Figure 1 is a schematic perspective view illustrating a first embodiment of a system for training visual acuity according to the disclosure;
Figure 2 is a block diagram illustrating the first embodiment of the system according to the disclosure;

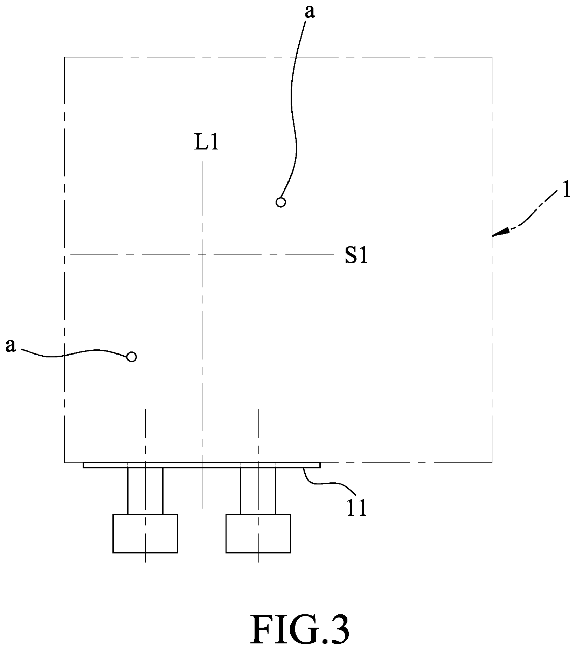

Figure 3 is a schematic diagram illustrating one view of a moving object in an embodiment of a motion picture generated by the system according to the disclosure;

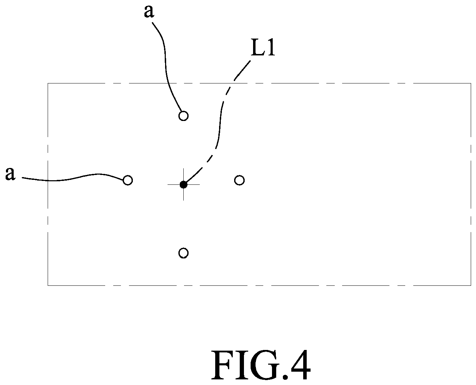

Figure 4 is a schematic diagram illustrating another view of an embodiment of the motion picture generated by the system according to the disclosure;
Figure 5 is a schematic diagram illustrating acupuncture points on a periorbital region of a user;
Figure 6 is a schematic diagram illustrating the first embodiment of the system in use by a user;
Figure 7 is a schematic diagram illustrating a result as to which brain region was stimulated based on an analysis of Magnetic Resonance Imaging (MRI) images of User No.1 who listened to monaural beats formed by two audio frequency components that have a frequency difference of 40 Hz;
Figure 8 is a schematic diagram illustrating a result as to which brain region was stimulated based on an analysis of MRI images of User No.2 who listened to the monaural beats formed by two audio frequency components that have a frequency difference of 40 Hz;
Figure 9 is a schematic diagram illustrating a result as to which brain region was stimulated based on an analysis of MRI images of User No.2 who listened to the monaural beats formed by two audio frequency components that have a frequency difference of 10 Hz;
Figure 10 is a schematic diagram illustrating a result as to which brain region was stimulated based on an analysis of MRI images of User No.2 who listened to the binaural beats formed by two audio frequency components that have a frequency difference of 10 Hz;
Figure 11 is a schematic diagram illustrating a result as to which brain region was stimulated based on an analysis of MRI images of User No.3 who listened to the monaural beats formed by two audio frequency components that have a frequency difference ranging from 4 to 7 Hz;
Figure 12 is a schematic diagram illustrating a result as to which brain region was stimulated based on an analysis of MRI images of User No.3 who listened to the monaural beats formed by two audio frequency components that have a frequency difference ranging from 8 to 11 Hz;
Figure 13 is a schematic diagram illustrating a result as to which brain region was stimulated based on an analysis of MRI images of User No.3 who listened to the monaural beats formed by two audio frequency components that have a frequency difference ranging from 12 to 15 Hz;
Figure 14 is a schematic diagram illustrating a result as to which brain region was stimulated based on an analysis of MRI images of User No.3 who listened to the monaural beats formed by two audio frequency components that have a frequency difference ranging from 20 to 23 Hz;
Figure 15 is a schematic diagram illustrating a result as to which brain region was stimulated based on an analysis of MRI images of User No.3 who listened to the monaural beats formed by two audio frequency components that have a frequency difference ranging from 24 to 27 Hz;
Figure 16 is a schematic perspective view illustrating a second embodiment of the system according to the disclosure;
Figure 17 is a schematic diagram illustrating the second embodiment of the system in use by a user;
Figure 18 is a block diagram illustrating the second embodiment of the system according to the disclosure;
Figure 19 is a schematic perspective view illustrating a third embodiment of the system according to the disclosure;
Figure 20 is a block diagram illustrating a fourth embodiment of the system according to the disclosure;
Figure 21 is a schematic diagram illustrating the fourth embodiment of the system mounted on a head of a user; and
Figure 22 is a schematic diagram illustrating a top view of the fourth embodiment of the system which is mounted on the head of the user.

Before the disclosure is described in greater detail, it should be noted that where considered appropriate, reference numerals or terminal portions of reference numerals have been repeated among the figures to indicate corresponding or analogous elements, which may optionally have similar characteristics.

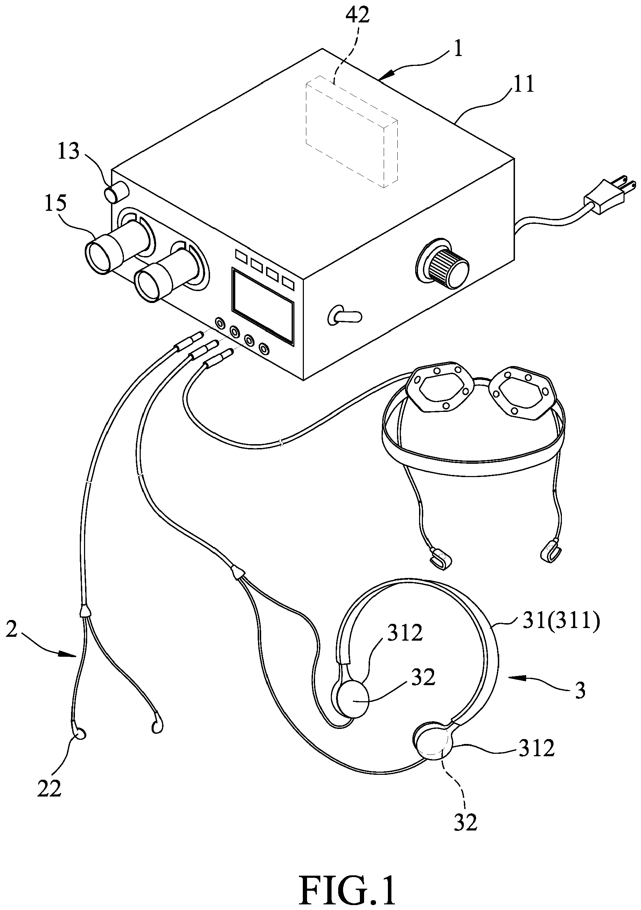

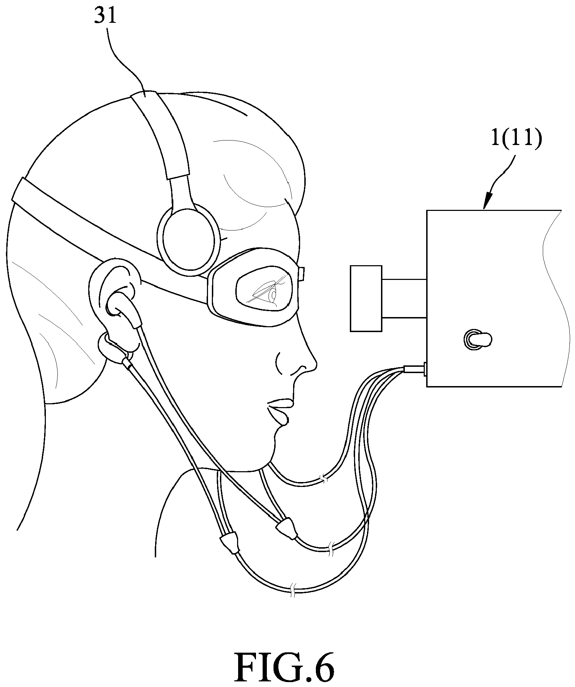

Referring to Figures 1, 2 and 6, a first embodiment of a system for training visual acuity is illustrated. The system includes an audio player 2, an electrical stimulator 3, a motion picture device 4, a controller 6, and a main console 1 to which the audio player 2, the motion picture device 4, the electrical stimulator 3 and the controller 6 are connected.

The main console 1 includes a housing 11, an input device 13 disposed on the housing 11 and configured to be operated by the user to input an operation instruction, and an observation device 15 disposed on the housing 11.

The audio player 2 includes a database 21 which stores plural pieces of music, and a headphone 22 with which a user listens to the pieces of music so as to stimulate a visual cortex of the user. The headphone 22 of the audio player 2 is disposed outside the housing 11.

Each of the pieces of music contains two audio frequency components that have a frequency difference therebetween ranging from 1 to 45 Hz for inducing the frequency following response (FFR) of the user. In this embodiment, each of the pieces of music may include monaural beats which are a mix of two different pure tones respectively formed by the audio frequency components, or binaural beats where the two pure tones respectively formed by the audio frequency components are to be respectively played to left and right ears of the user.

The electrical stimulator 3 is configured to generate and regulate a low frequency electric current by a current-control component (not shown) which is disposed on the main console 1, where the frequency of the low frequency electric current thus generated is set by referring to a general product specification of a transcutaneous electrical nerve stimulator on the market. In some embodiments, the current-control component is disposed outside the main console 1. The electrical stimulator 3 includes two first electrodes 32 via which the low frequency electric current ranging between 0 and 100 micro-Amperes is to be outputted. The first electrodes 32 are disposed outside the main console 1, are electrically connected to the current-control component, and are configured to be respectively attached to temples or cheeks of the user. The electrical stimulator 3 further includes an arc frame 31 having two end portions 312 and a bridge portion 311 that interconnects the end portions 312. The end portions 312 each have an inner side. The inner sides of the end portions 312 face each other and the first electrodes 32 are respectively connected to the inner sides. The end portions 312 are connected telescopically to the bridge portion 311 so as to position the first electrodes 32 respectively at the temples or the cheeks of the user when the electrical stimulator 3 is mounted on a head of the user. In this embodiment, the arc frame 31 is made of flexible material.

In a scenario of using the electrical stimulator 3, the user is able to wear the electrical stimulator 3 by mounting the arc frame 31 on his/her head, and adjusting the end portions 312 to position the first electrodes 32 respectively at the temples of the user. Then, a magnitude of the low frequency electric current outputted by the electrical stimulator 3 is appropriately regulated for stimulating nerves in periocular regions of the user, relaxing ocular muscles of the user and improving blood circulation in periocular regions of the user. Moreover, the user is able to perform eye movement and eye tracking to enhance the effect of training.

The motion picture device 4 is disposed on the main console 1, and is configured to generate a motion picture for guiding eye movement of the user. The motion picture device 4 includes a display 42 that is disposed in the housing 11 and that is configured to display the motion picture generated by the motion picture device 4 so as to enable the user to watch the motion picture through the observation device 15. The display 42 may be implemented to be a digital photo frame. The motion picture may be presented as a light spot or any light pattern that is movable. In some embodiments, the motion picture device 4 may be implemented to generate the motion picture by an array of light bulbs (not shown) disposed in the housing 11. The light bulbs are programmed to be turned on or off in a predetermined order so as to result in the motion picture. However, implementation of the motion picture is not limited to the disclosure herein and may vary in other embodiments . In one embodiment, the motion picture device 4 may be implemented to be a virtual reality (VR) device. The motion picture may be a scene of a moving object over a background, e.g., an airplane flying in the sky or a sailboat sailing the ocean. Optionally, the moving object in the motion picture may be user controlled using a controller or a keyboard for promoting user interaction and providing entertainment. Through eye movement under guidance of the moving object in the motion picture provided by the motion picture device 4, ciliary muscles and extraocular muscles of the user are trained and relaxed.

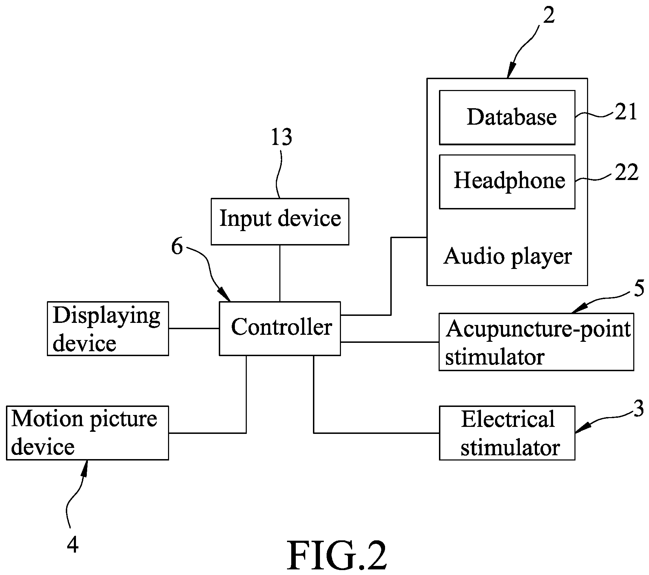

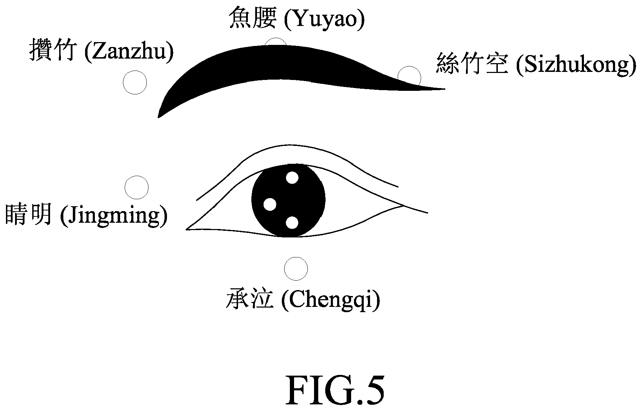

In one embodiment, the system for training visual acuity further include an acupuncture-point stimulator 5 (see Figure 2) configured to stimulate acupuncture points shown in Figure 5.

The controller 6 is disposed in the housing 11. The controller 6 is electrically connected to the input device 13, the audio player 2, the electrical stimulator 3 and the motion picture device 4. The controller 6 is configured to receive the operation instruction from the input device 13 so as to control operation of one or more of the audio player 2, the electrical stimulator 3 and the motion picture device 4. Specifically speaking, in response to the operation instruction inputted via the input device 13, the controller 6 is configured to control the audio player 2 to select and play one of the pieces of music, to control the electrical stimulator 3 to regulate the magnitude of the low frequency electric current outputted by the electrical stimulator 3 and to activate or deactivate the motion picture device 4.

In use, the user is capable of wearing the headphone 22 to listen to the pieces of music stored in the database 21. Any individual one of the pieces of music has a specific frequency difference between the audio frequency components so as to induce a brain wave of the user that has a specific wavelength, and to activate and regulate activities of brain regions involved in visual information processing. While listening to the music, the user is able to simultaneously utilize the electrical stimulator 3 to stimulate his/her temples. Moreover, by utilizing the motion picture device 4 to watch the motion picture generated thereby via the observation device 15 at the same time, the user is able to exercise eye movement and eye tracking. The above-mentioned training may continue for a duration ranging from 20 to 30 minutes to train ocular muscles. Since brain regions, nerves and muscles that are related to visual perception are trained at the same time, an effect of improving visual acuity may be greatly enhanced by the system according to the disclosure.

According to the result of an experiment conducted by the inventors, a piece of music containing two audio frequency components that have a frequency difference therebetween ranging from 1 to 45 Hz can be utilized to effectively stimulate the visual cortex in an occipital lobe of the user. The experiment is based on the disclosure of Taiwanese Invention Patent No.I543749 and includes steps described below.

In step 1, plural pieces of music were prepared, where each of the pieces of music corresponded to a specific frequency difference of audio frequency components contained in the piece of music. Each of the pieces of music was implemented to contain monaural beats or binaural beats, and served as a piece of test music. In addition, another piece of music that contained no monaural beats or binaural beasts was prepared, and served as a piece of reference music.

In step 2, multiple users were initially requested to listen to the piece of reference music for one minute, and brain activities of the users were recorded as reference brain-activity data based on Magnetic Resonance Imaging (MRI) images. Subsequently, the users were requested to listen to one of the pieces of test music for one minute, and brain activities of the users were recorded as test brain-activity data, based similarly on MRI images.

In step S3, brain regions activated by listening to the one of the pieces of test music were determined based on a comparison between the reference brain-activity data and the test brain-activity data that respectively corresponded to the piece of reference music and the one of the pieces of test music.

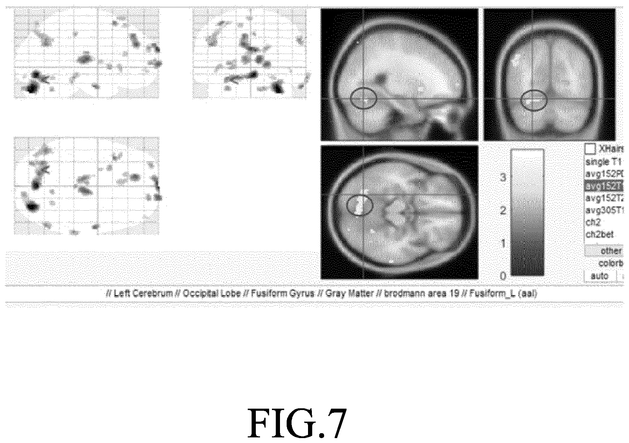

Figures 7 to 15 illustrate results of the determination as to which brain regions were stimulated and activated based on analyses of MRI images of three users (hereinafter referred to as User No.1, User No.2 and User No. 3) who listened to the pieces of test music played by the system according to this disclosure for one minute. The activated brain regions are indicated by circles and each of the pieces of test music was formed by two audio frequency components with a frequency difference ranging from 1 to 40 Hz.

Referring to Figure 7, a result of an analysis of MRI images of User No.1 who listened to the piece of test music which contained monaural beats formed by two audio frequency components with a frequency difference of 40 Hz is illustrated. The result shows that a visual cortex in an occipital lobe of User No.1 was stimulated and activated.

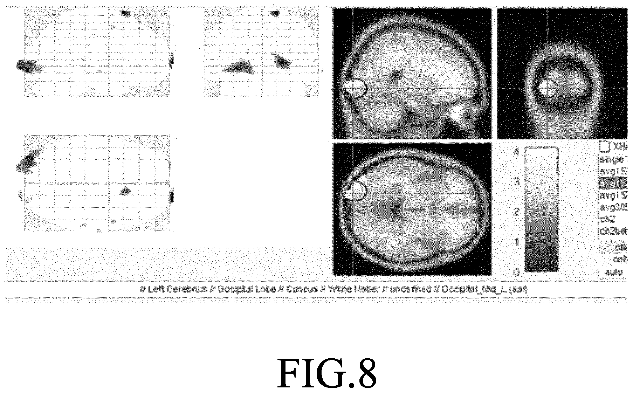

Referring to Figure 8, a result of an analysis of MRI images of User No.2 who listened to the piece of test music which contained monaural beats formed by two audio frequency components with a frequency difference of 40 Hz is illustrated. The result shows that a visual cortex in an occipital lobe of User No.2 was stimulated and activated.

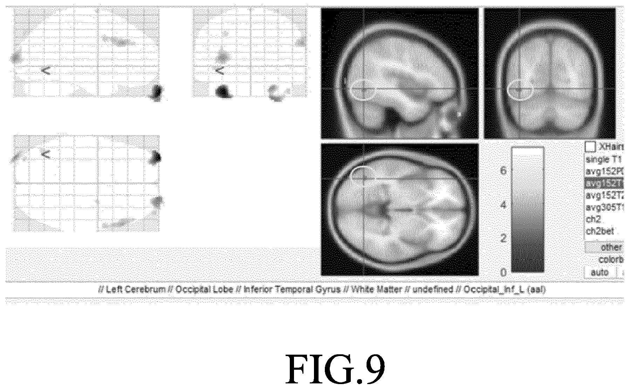

Referring to Figure 9, a result of an analysis of MRI images of User No.2 who listened to the piece of test music which contained monaural beats formed by two audio frequency components with a frequency difference of 10 Hz is illustrated. The result shows that the visual cortex in the occipital lobe of User No.2 was stimulated and activated.

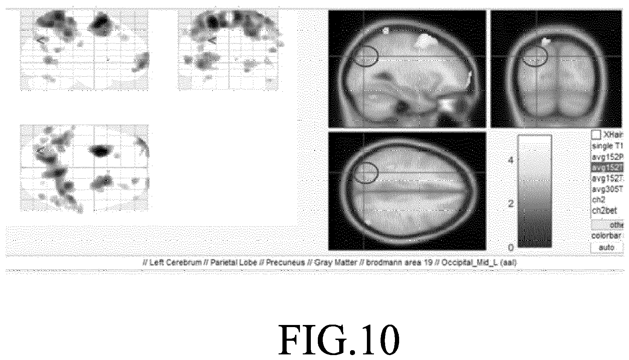

Referring to Figure 10, a result of an analysis of MRI images of User No.2 who listened to the piece of test music which contained binaural beats formed by two audio frequency components with a frequency difference of 10 Hz is illustrated. The result shows that the visual cortex in the occipital lobe of User No.2 was stimulated and activated.

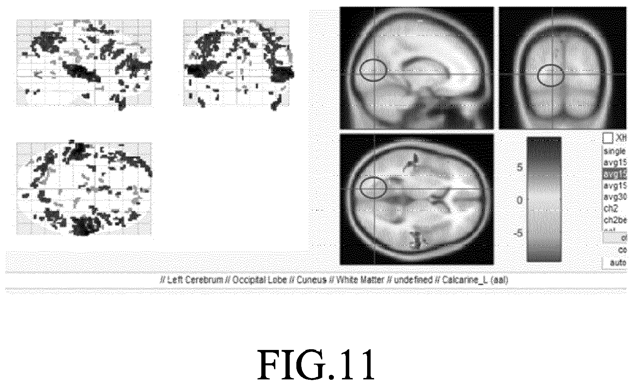

Referring to Figure 11, a result of an analysis of MRI images of User No.3 who listened to the piece of test music which contained monaural beats formed by two audio frequency components with a frequency difference ranging from 4 to 7 Hz is illustrated. The result shows that a visual cortex in an occipital lobe of User No.3 was stimulated and activated.

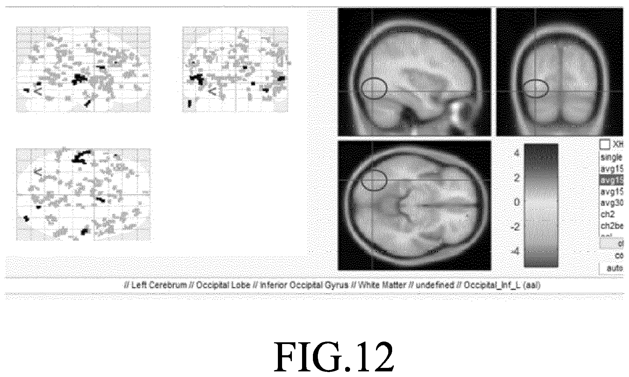

Referring to Figure 12, a result of an analysis of MRI images of User No.3 who listened to the piece of test music which contained monaural beats formed by two audio frequency components with a frequency difference ranging from 8 to 11 Hz is illustrated. The result shows that the visual cortex in the occipital lobe of User No.3 was stimulated and activated.

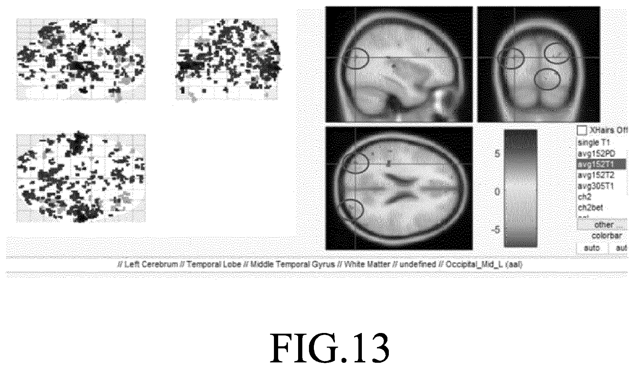

Referring to Figure 13, a result of an analysis of MRI images of User No.3 who listened to the piece of test music which contained monaural beats formed by two audio frequency components with a frequency difference ranging from 12 to 15 Hz is illustrated. The result shows that the visual cortex in the occipital lobe of User No.3 was stimulated and activated.

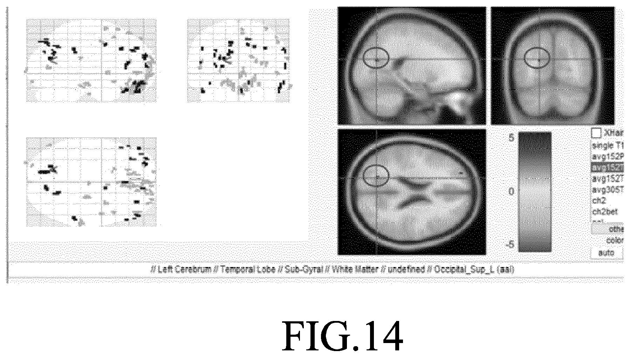

Referring to Figure 14, a result of an analysis of MRI images of User No.3 who listened to the piece of test music which contained monaural beats formed by two audio frequency components with a frequency difference ranging from 20 to 23 Hz is illustrated. The result shows that the visual cortex in the occipital lobe of User No.3 was stimulated and activated.

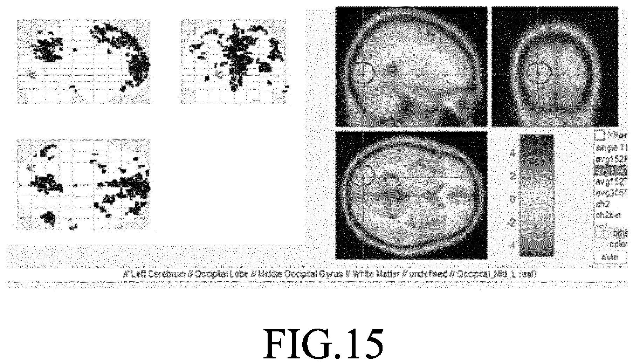

Referring to Figure 15, a result of an analysis of MRI images of User No.3 who listened to the piece of test music which contained monaural beats formed by two audio frequency components with a frequency difference ranging from 24 to 27 Hz is illustrated. The result shows that the visual cortex in the occipital lobe of User No.3 was stimulated and activated.

Referring to Table 1 below, a result of vision tests performed on ten users with the naked eye before and after utilizing the system according to the disclosure is shown. Within a span of two weeks, each of the users utilized the system according to the disclosure more than once, and each usage period lasted 30 minutes . Each of the users simultaneously utilized the motion picture device 4 to perform eye movement and eye tracking, listened to the pieces of music played by the audio player 2, and utilized the electrical stimulator 3 to stimulate his/her temples. The fifth column of Table 1 shows respective visual acuities of right and left eyes of the users with the naked eye prior to the first time he/she started to utilize the system according to the disclosure, and the sixth column of Table 1 shows respective visual acuities of right and left eyes of the users with the naked eye after the last time within the two weeks he/she utilized the system according to the disclosure. The result provided by Table 1 shows that the visual acuities of the ten users were apparently improved over the two weeks. Consequently, it is proven that the system according to the disclosure is capable of enhancing visual acuity of a user by utilizing the audio player 2 to play music which contains two audio frequency components with a frequency difference therebetween to stimulate and activate a visual cortex of the user, by utilizing the electrical stimulator 3 to apply a low frequency electric current to the user so as to improve blood circulation and stimulate muscles in periocular regions of the user, and by utilizing the motion picture device 4 to generate a motion picture to guide the eye movement and eye tracking of the user, simultaneously.

**Table 1**

| User number | Counts of usage in two weeks | Duration (Minute) | Eye | Visual acuity before use | Visual acuity after use |
|---|---|---|---|---|---|
| 1 | > 1 | 30 | R | 0.6 | 0.7 |
| | | | L | 0.9 | 1.0 |
| 2 | > 1 | 30 | R | 0.2 | 0.2 |
| | | | L | 0.1 | 0.1 |
| 3 | > 1 | 30 | R | 0.1 | 0.2 |
| | | | L | 0 . 1 | 0.2 |
| 4 | > 1 | 30 | R | 0.1 | 0.2 |
| | | | L | 0.2 | 0.2 |
| 5 | > 1 | 30 | R | 1.2 | 1.5 |
| | | | L | 1.2 | 1.5 |
| 6 | > 1 | 30 | R | 0.3 | 0.5 |
| | | | L | 0.2 | 0.3 |
| 7 | > 1 | 30 | R | 0.3 | 0.6 |
| | | | L | 0.6 | 0.6 |
| 8 | > 1 | 30 | R | 0.9 | 1.2 |
| | | | L | 1.0 | 1.2 |
| 9 | > 1 | 30 | R | 0.6 | 0.7 |
| | | | L | 0.9 | 1.0 |
| 10 | > 1 | 30 | R | 0.1 | 0.1 |
| | | | L | 0.1 | 0.1 |

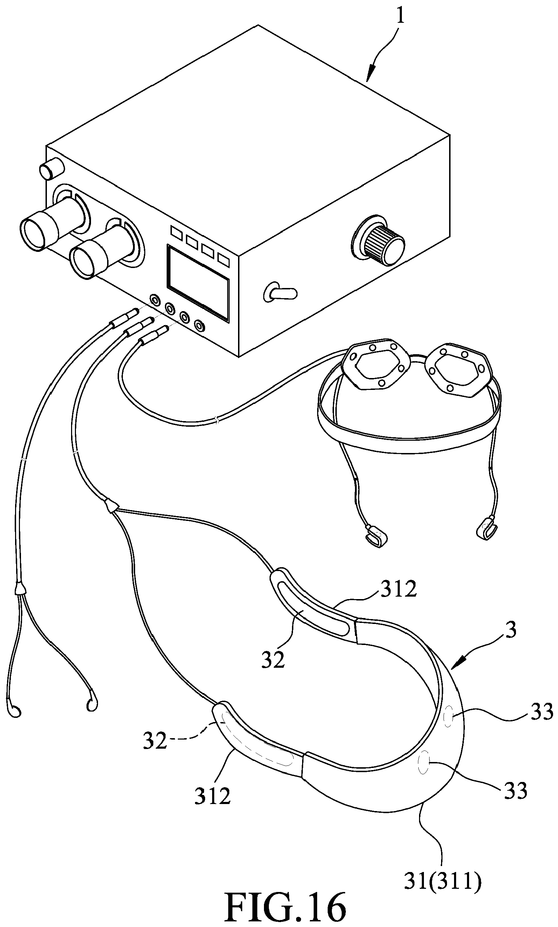

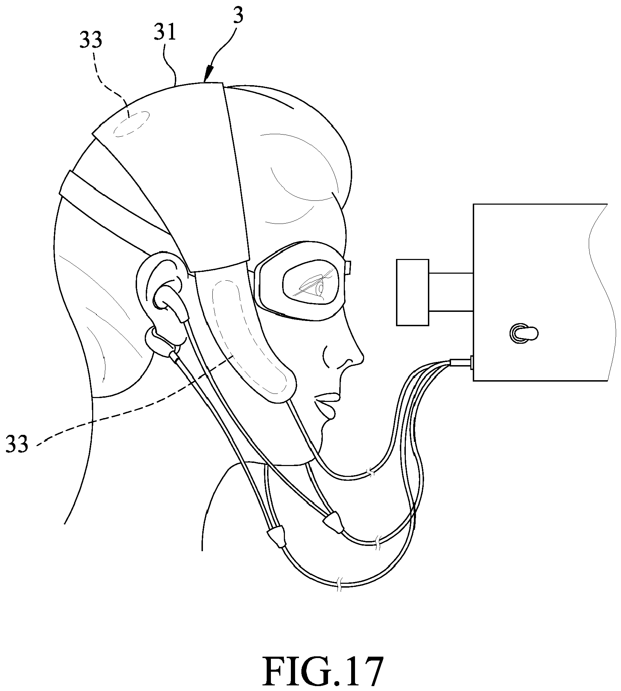

Referring to Figures 16 and 17, a second embodiment of the system according to the disclosure is illustrated. The second embodiment is similar to the first embodiment, but is different from the first embodiment in what are described as follows.

Dimensions of the first electrodes 32 of the electrical stimulator 3 in the second embodiment are greater than those of the first electrodes 32 in the first embodiment so that areas from temples to cheeks of the user may be covered by the first electrodes 32. The bridge portion 311 of the arc frame 31 of the electrical stimulator 3 of the second embodiment is wider. The electrical stimulator 3 of the second embodiment further includes two second electrodes 33 disposed on the bridge portion 311 of the arc frame 31 and spaced apart from each other along a longitudinal direction of the bridge portion 311.

The controller 6 (see Figure 2) of the second embodiment is configured to, based on the operation instruction, control the electrical stimulator 3 to switch to one of a transcutaneous electric nerve stimulation (TENS) mode, a first transcranial direct current stimulation (tDCS) mode and a second tDCS mode so as to apply a low frequency electric current to the temples and cheeks of the user or a direct current (DC) electric current to the visual cortex of the user.

The electrical stimulator 3 of the second embodiment is configured to, when in the TENS mode, perform TENS on the user with a low frequency electric current which ranges from 0 to 100 milli-Amperes via the first electrodes 32. In the TENS mode, the second electrodes 33 do not conduct electricity. The electrical stimulator 3 of the second embodiment is configured to, when in the first tDCS mode, perform tDCS on the user with a DC electric current which ranges from 0 to 10 milli-Amperes via one of the first electrodes 32 and one of the second electrodes 33 which respectively serve as the cathode and the anode and which are located on different sides of the user. The electrical stimulator 3 of the second embodiment is configured to, when in the second tDCS mode, perform tDCS on the user with a DC electric current which ranges from 0 to 10 milli-Amperes via the other one of the first electrodes 32 and the other one of the second electrodes 33 which respectively serve as the cathode and the anode and which are located on different sides of the user.

In the second embodiment, locations of the second electrodes 33 on the arc frame 31 respectively correspond to locations "P1" and "P2" in the international 10-20 system that describes locations on a subject for disposing electrodes. In one embodiment, the electrical stimulator 3 is configured to, when in the first tDCS mode or the second tDCS mode, perform tDCS on the user with a DC electric current which ranges from 0 to 2.5 milli-Amperes. Based on practical experience, eye tracking ability of the user is greatly improved when the DC electric current applied to the user is 2 milli-Amperes.

In a scenario of using the electrical stimulator 3 in the first tDCS mode or the second tDCS mode, the user is able to wear the electrical stimulator 3 by mounting the arc frame 31 on his/her head, and adjusting the end portions 312 so as to position the first electrodes 32 respectively at the areas covering his/her temples and cheeks, and to position the second electrodes 33 respectively at the above-mentioned "P1" and "P2" locations. After that, the user switches the electrical stimulator 3 to one of the first tDCS mode and the second tDCS mode. Then, a magnitude of the DC electric current outputted by the electrical stimulator 3 is gradually adjusted from 0 milli-Amperes to an appropriate value. Besides the effects achieved by the electrical stimulator 3 in the first embodiment, the electrical stimulator 3 in the second embodiment further stimulates the visual cortex in occipital lobe of the user for visual acuity enhancement.

In one embodiment, a duration of using the electrical stimulator 3 in each of the TENS mode, the first tDCS mode and the second tDCS mode is 20 minutes.

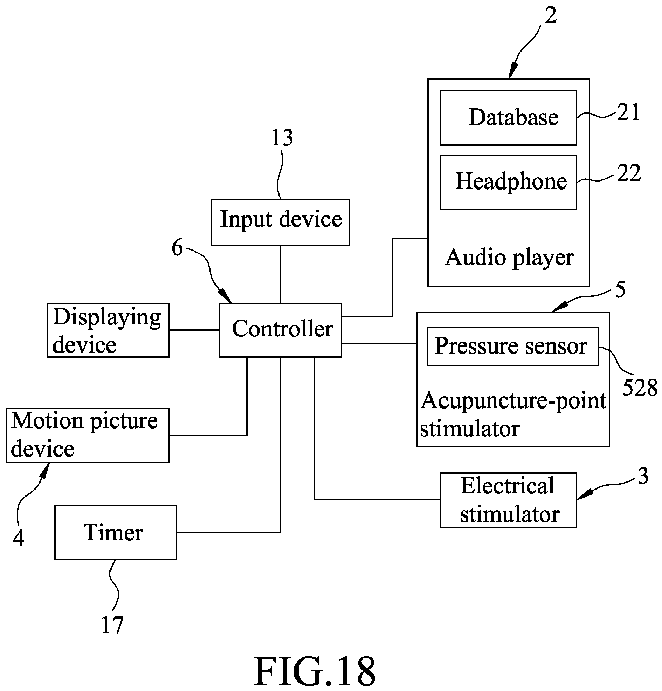

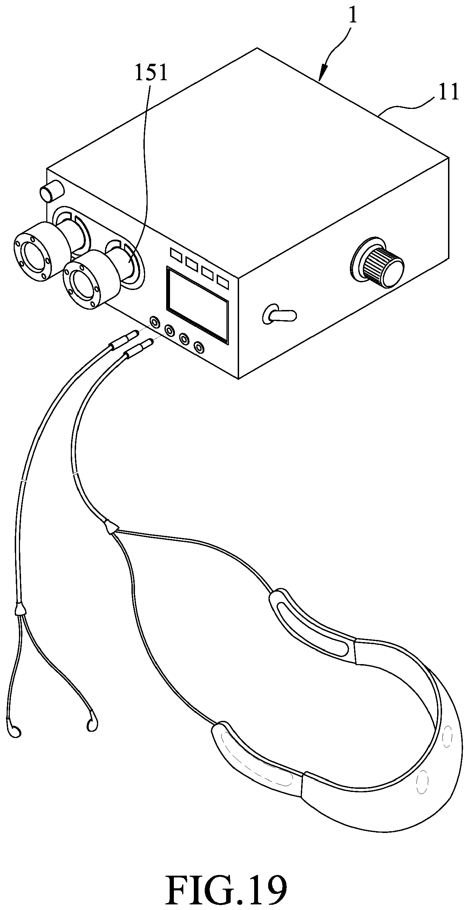

Referring to Figures 18 and 19, a third embodiment of the system according to the disclosure is illustrated. In this embodiment, the system further includes an acupuncture stimulator 5 that includes a pressure sensor 528.

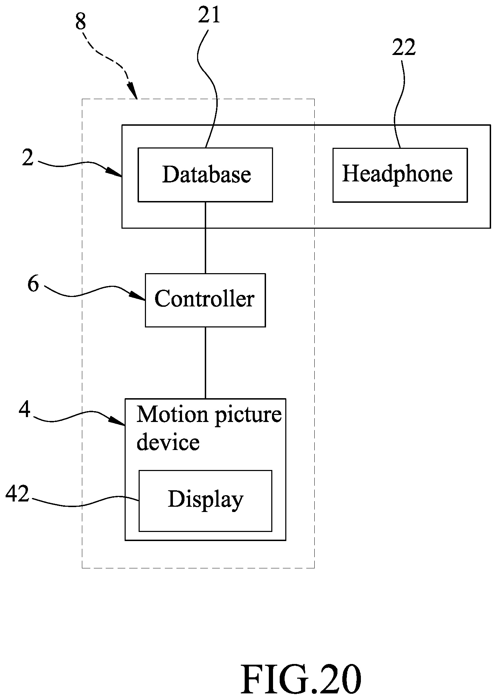

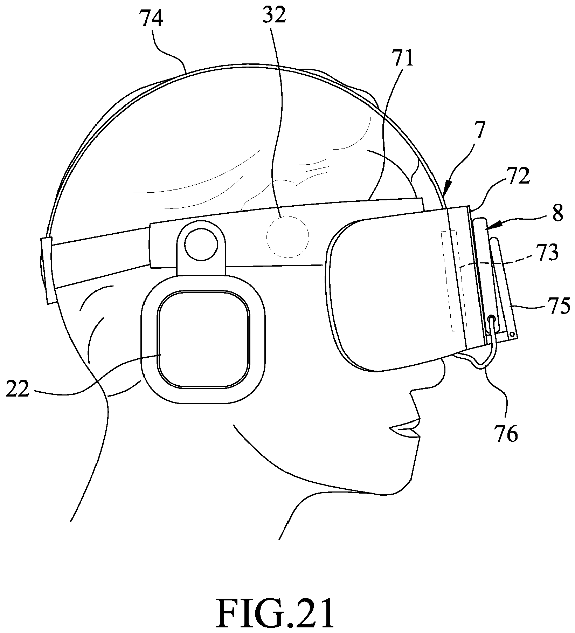

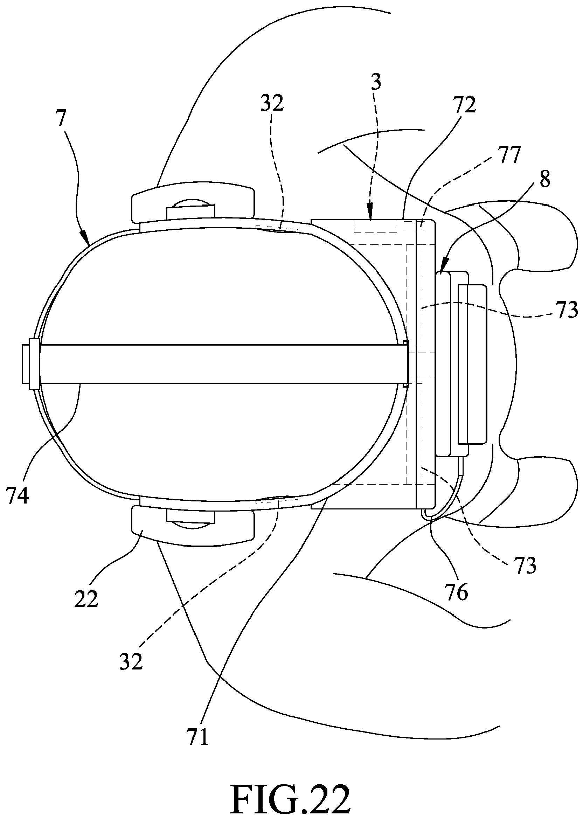

Referring to Figures 20 to 22, a fourth embodiment of the system according to the disclosure is illustrated. The fourth embodiment is similar to the first embodiment, but is different from the first embodiment in what are described as follows.

The fourth embodiment of the system does not include the main console 1. The fourth embodiment of the system further includes a wearable device 7 configured to be mounted on a head of the user, and a portable electronic multimedia device 8.

The portable electronic multimedia device 8 includes the database 21 of the audio player 2, the motion picture device 4 and the controller 6. The motion picture device 4 includes a display 42 that is configured to display the motion picture generated by the motion picture device 4. In this embodiment, the portable electronic multimedia device 8 is implemented to be a smartphone. The controller 6 is implemented to be a central processing unit (CPU) and a circuit board of the smartphone. The database 21 is implemented to be stored in a memory of the smartphone, and the memory further stores data utilized by the motion picture device 4 for generating the motion picture. The display 42 of the motion picture device 4 is implemented to be a screen of the smartphone. However, implementations of the database 21, the motion picture device 4 and the controller 6 by the portable electronic multimedia device 8 are not limited to the disclosure herein and may vary in other embodiments.

The wearable device 7 includes a U-shaped frame 71, an outer frame 72, two lens sets 73, a fastening band 74 and a holder 75. The U-shaped frame 71 is configured to be mounted on a forehead of the user. The outer frame 72 is disposed on an outer side of the U-shaped frame 71 and is configured to abut against periorbital regions of the user. The lens sets 73 are disposed on an inner side of the outer frame 72. The lens sets 73 respectively correspond to eyes of the user, and are configured to enable the motion picture generated by the motion picture device 4 to be watched in a stereoscopic manner. The fastening band 74 is connected to the U-shaped frame 71, and is configured to mount the U-shaped frame 71 on the head of the user.

By the holder 75, the portable electronic multimedia device 8 is removably disposed on an outer side of the outer frame 72 of the wearable device 7 opposite to the inner side of the outer frame 72. The headphone 22 of the audio player 2 is removably connected to the portable electronic multimedia device 8 via a cable 76, and is disposed on the U-shaped frame 71.

The electrical stimulator 3 is disposed on the wearable device 7, and is electrically connected to an accumulator battery 77 disposed in the wearable device 7. The first electrodes 32 of the electrical stimulator 3 are disposed on an inner side of the U-shaped frame 71 that is opposite to the outer side of the U-shaped frame 71. The first electrodes 32 of the electrical stimulator 3 respectively correspond to the temples of the user.

Besides the effects achieved by the system in the first embodiment, the system of the fourth embodiment further has the following advantages. The system according to the fourth embodiment can be mounted on the head of the user with the assistance of the wearable device 7, so the user is able to take any comfortable posture (e.g., sitting or lying down) when using the system. Therefore, comfort and convenience of using the system are enhanced. In addition, the portable electronic multimedia device 8 is implemented by a general mobile phone or a tablet computer, so convenience of using the system is further enhanced. Moreover, the lens sets 73 enable the motion picture generated by the system to be watched in a stereoscopic manner, providing the user with more entertaining experience when using the system.

In summary, the system for training visual acuity according to the disclosure stimulates the visual cortex of the user by utilizing the audio player 2 to play one or more pieces of music each of which contains two audio frequency components with a frequency difference therebetween ranging from 1 to 45 Hz to be heard by the user. By utilizing the electrical stimulator 3 to apply an electric current ranging between 0 and 100 micro-Amperes to the temples or the cheeks of the user, nerves in periocular regions of the user may be stimulated, ocular muscles of the user may be relaxed, and blood circulation in the periocular regions of the user may be improved. By utilizing the motion picture device 4 to generate a motion picture to guide the eye movement and eye tracking of the user, the user is able to train visual acuity by doing eye exercises. Last but not least, by simultaneously utilizing the audio player 2 and the electrical stimulator 3 during eye exercising, the visual acuity of the user can be trained and enhanced with higher efficiency and effectiveness.

In the description above, for the purposes of explanation, numerous specific details have been set forth in order to provide a thorough understanding of the embodiments. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects, and that one or more features or specific details from one embodiment may be practiced together with one or more features or specific details from another embodiment, where appropriate, in the practice of the disclosure.

## Claims

1. A system for training visual acuity, said system **characterized by**:
an audio player (2) including a database (21) which stores plural pieces of music, and a headphone (22) with which a user listens to the pieces of music so as to stimulate a visual cortex of the user, where each of the pieces of music contains two audio frequency components that have a frequency difference therebetween ranging from 1 to 45 Hz; and
a controller (6) electrically connected to said audio player (2), and configured to control said audio player (2) to play the pieces of music.

2. The system as claimed in claim 1, further **characterized by** an electrical stimulator (3) that includes two first electrodes (32) via which an electric current ranging between 0 and 100 micro-Amperes is to be outputted, said first electrodes (32) being configured to be respectively attached to temples or cheeks of the user.

3. The system as claimed in claim 2, further **characterized by**:
a motion picture device (4) configured to generate a motion picture for guiding eye movement of the user,
in that said controller (6) is electrically connected to said motion picture device (4), and is configured to activate or deactivate said motion picture device (4).

4. The system as claimed in claim 3, further **characterized by**:
a main console (1) to which said audio player (2), said motion picture device (4), said electrical stimulator (3) and said controller (6) are connected,
in that said main console (1) includes
a housing (11),
an input device (13) disposed on said housing (11) and configured to be operated by the user to input an operation instruction, and
an observation device (15) disposed on said housing (11),
in that said motion picture device (4) includes a display (42) that is disposed in said housing (11) and that is configured to display the motion picture generated by said motion picture device (4) so as to enable the user to watch the motion picture through said observation device (15), and
in that said controller (6) is disposed in said housing (11), is electrically connected to said input device (13) and said electrical stimulator (3), and is configured to receive the operation instruction so as to control operation of one or more of said audio player (2), said motion picture device (4) and said electrical stimulator (3).

5. The system as claimed in claim 4, **characterized in that** said electrical stimulator (3) further includes an arc frame (31) having:
two end portions (312) each having an inner side, said inner sides of said end portions (312) facing each other and being connected to said first electrodes (32); and
a bridge portion (311) that interconnects said end portions (312), said end portions (312) being connected telescopically to said bridge portion (311) so as to position said first electrodes (32) respectively at the temples or the cheeks of the user when said electrical stimulator (3) is mounted on a head of the user.

6. The system as claimed in claim 5, **characterized in that**:
said controller (6) is configured to, based on the operation instruction, control said electrical stimulator (3) to switch to one of a transcutaneous electric nerve stimulation, TENS, mode, a first transcranial direct current stimulation, tDCS, mode and a second tDCS mode; and
said electrical stimulator (3) further includes two second electrodes (33) disposed on said bridge portion (311) of said arc frame (31) and spaced apart from each other along a longitudinal direction of said bridge portion (311), and is configured to,
when said electrical stimulator (3) is in the TENS mode, perform TENS on the user with an electric current which ranges between 0 and 100 milli-Amperes via said first electrodes (32),
when said electrical stimulator (3) is in the first tDCS mode, perform tDCS on the user with a direct current, DC, electric current which ranges between 0 and 10 milli-Amperes via one of said first electrodes (32) and one of said second electrodes (33) which respectively serve as a cathode and an anode and which are located on different sides of the user, and
when said electrical stimulator (3) is in the second tDCS mode, perform tDCS on the user with a DC electric current which ranges between 0 and 10 milli-Amperes via the other one of said first electrodes (32) and the other one of said second electrodes (33) which respectively serve as the cathode and the anode and which are located on the different sides of the user.

7. The system as claimed in claim 3, further **characterized by**:
a wearable device (7) configured to be mounted on a head of the user, and including
a U-shaped frame (71) that is configured to be mounted on a forehead of the user,
an outer frame (72) that is disposed on an outer side of said U-shaped frame (71) and that is configured to abut against periorbital regions of the user,
two lens sets (73) that are disposed on an inner side of said outer frame (72), that respectively correspond to eyes of the user, and that are configured to enable the motion picture generated by said motion picture device (4) to be watched in a stereoscopic manner, and
a fastening band (74) that is connected to said U-shaped frame (71) and that is configured to mount said U-shaped frame (71) on the head of the user; and
a portable electronic multimedia device (8) removably disposed on an outer side of said outer frame (72) of said wearable device (7) opposite to said inner side of said outer frame (72), and including said database (21) of said audio player (2), said motion picture device (4) and said controller (6),
in that said electrical stimulator (3) is disposed on said wearable device (7), said first electrodes (32) of said electrical stimulator (3) being disposed on an inner side of said U-shaped frame (71) that is opposite to said outer side of said U-shaped frame (71), and respectively corresponding to the temples of the user,
in that said motion picture device (4) includes a display (42) that is configured to display the motion picture generated by said motion picture device (4),
in that said headphone (22) of said audio player (2) is removably connected to said portable electronic multimedia device (8).

8. The system as claimed in any one of claims 1 to 7, **characterized in that** each of the pieces of music includes monaural beats which are a mix of two different pure tones respectively formed by the audio frequency components.
